(19) Europäisches Patentamt European Patent Office Office européen des brevets

(11) **EP 4 491 366 A1**

(12) **EUROPEAN PATENT APPLICATION**

(43) Date of publication:
**15.01.2025 Bulletin 2025/03**

(21) Application number: **24185264.9**

(22) Date of filing: **28.06.2024**

(51) International Patent Classification (IPC):
**B29C 45/00** (2006.01)      **G01N 3/08** (2006.01)
**G01N 33/44** (2006.01)      **B29L 31/40** (2006.01)

(52) Cooperative Patent Classification (CPC):
**B29C 45/00; G01N 33/442; B29L 2031/40**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **14.07.2023  JP 2023115781**

(71) Applicant: **Ricoh Company, Ltd.**
**Tokyo 143-8555 (JP)**

(72) Inventor: **UEDA, Kenji**
**Tokyo, 143-8555 (JP)**

(74) Representative: **J A Kemp LLP**
**80 Turnmill Street**
**London EC1M 5QU (GB)**

(54) **RESIN MOLDED ARTICLE AND DETERMINING METHOD**

(57)    A resin molded article (10) includes multiple beam-shaped portions (20A to 20D) each having a fixed end connected to a bottom of the resin molded article (10) and a free end rising from the bottom of the resin molded article in a first direction, the free end plastically deformable. The multiple beam-shaped portions (20A to 20D) have different maximum bending stresses in bending the free end by a predetermined amount in a second direction intersecting the first direction.

## Description

BACKGROUND

Technical Field

Embodiments of the present disclosure relate to a resin molded article made of a predetermined resin material such as a component of an apparatus, and a method for determining whether the resin molded article has deteriorated.

Related Art

In the related art, in various apparatuses (e.g., industrial products), resin molded articles (i.e., resin parts) made of a predetermined resin material have been used as the component parts (e.g., Japanese Unexamined Patent Application Publication No. 2001-180782 and Japanese and Japanese Unexamined Patent Application Publication No. 2013-29371). After such a resin molded product has finished its service in the market and is collected from the market, the degree of deterioration is determined in a recycling factory, and the possibility of reuse is determined according to the degree of deterioration.

On the other hand, Japanese Unexamined Patent Application Publication No. 2001-180782 discloses a technique that measures a breaking force when a hole is made in a semiconductor component storage tray using a measuring device, and determines whether the storage tray can be reused from the measurement result. In addition, Japanese Unexamined Patent Application Publication No. 2013-29371 discloses a technique that determines the degree of thermal degradation of a molded product by measuring the amount of an index component when a pin-shaped protrusion disposed on the molded product is cut off and performing a differential scanning calorimetry (DSC) analysis by a calorimeter to evaluate the thermal history.

In the related art, since whether the resin molded article has deteriorated is determined by a measuring device, it takes time and effort.

An object of the present disclosure is to solve the problems described above and to provide a resin molded article and determining method for deterioration of the resin molded article.

SUMMARY

According to an embodiment of the present disclosure, a resin molded article includes multiple beam-shaped portions each having a fixed end connected to a bottom of the resin molded article, and a free end rising from the bottom of the resin molded article in a first direction, the free end plastically deformable. The multiple beam-shaped portions have different maximum bending stresses in bending the free end by a predetermined amount in a second direction intersecting the first direction.

According to an embodiment of the present disclosure, a determining method includes deforming a free end of each of multiple beam-shaped portions of a resin molded article. The free end of each of the multiple beam-shaped portions is plastically deformable or breakable. The multiple beam-shaped portions have combinations of form of plastically deformed, broken, plastically undeformed, and unbroken when the free end is deformed by a predetermined amount.

According to embodiments of the present disclosure, a resin molded article and a determining method for the deterioration of the resin molded article in a simple manner can be provided.

BRIEF DESCRIPTION OF THE DRAWINGS

A more complete appreciation of embodiments of the present disclosure and many of the attendant advantages and features thereof can be readily obtained and understood from the following detailed description with reference to the accompanying drawings, wherein:

FIG. 1 is a perspective view of an apparatus according to an embodiment of the present disclosure;
FIG. 2 is a perspective view of a resin molded article according to an embodiment of the present disclosure;
FIG. 3 is a perspective view of multiple beam-shaped portions of the resin molded article in FIG. 2;
FIG. 4 is a top view of the multiple beam-shaped portion and its surroundings in FIG. 5;
FIG. 5 is a cross-sectional side view of a deformed beam-shaped portion of the multiple beam-shaped portions;
FIG. 6A is a graph of the relation between stress and strain in a beam-shaped portion without deterioration;
FIG. 6B is a graph of the relation between stress and strain in a beam-shaped portion having deteriorated;
FIG. 7 is a perspective view of the multiple beam-shaped portions and their surroundings according to a first modification; and
FIG. 8 is a perspective view of multiple sets of the multiple beam-shaped portions and its surroundings according to a second modification.

The accompanying drawings are intended to depict embodiments of the present disclosure and should not be interpreted to limit the scope thereof. The accompanying drawings are not to be considered as drawn to scale unless explicitly noted. Also, identical or similar reference numerals designate identical or similar components throughout the several views.

# DETAILED DESCRIPTION

In describing embodiments illustrated in the drawings, specific terminology is employed for the sake of clarity. However, the disclosure of this specification is not intended to be limited to the specific terminology so selected and it is to be understood that each specific element includes all technical equivalents that have a similar function, operate in a similar manner, and achieve a similar result.

Referring now to the drawings, embodiments of the present disclosure are described below. As used herein, the singular forms "a," "an," and "the" are intended to include the plural forms as well, unless the context clearly indicates otherwise.

According to embodiments of the present disclosure, a resin molded article and a determining method for the deterioration of the resin molded article in a simple manner can be provided.

Embodiments of the present disclosure are described in detail with reference to the drawings. In the drawings, the same or like reference signs denote like elements having substantially the same or corresponding configurations, and descriptions thereof may be omitted.

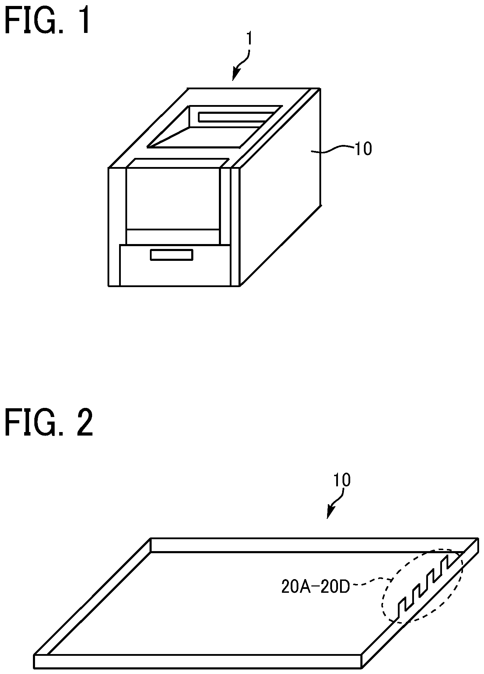

As illustrated in FIG. 1, an apparatus 1 includes a resin molded article 10 that is reusable. The resin molded article 10 is made of a predetermined resin material (e.g., polycarbonate (PC) resin, polyethylene terephthalate (PET) resin, or acrylonitrile butadiene styrene (ABS) resin). After the apparatus 1 has finished its service in the market, the apparatus 1 is collected from the market. The apparatus 1 is disassembled in a recycling factory to extract the resin molded article 10, and the degree of deterioration of the resin molded article 10 is determined. The reuse of the resin molded article 10 is determined depending on the degree of deterioration.

In the present embodiment, an image forming apparatus such as a printer is used as the apparatus 1. However, the apparatus 1 is not limited to the image forming apparatus, and any industrial product can be an object. In the present embodiment, a resin exterior cover is used as the resin molded article 10 that is reusable. However, the resin molded article 10 that is reusable is not limited to the resin exterior cover, and any resin molded article can be an object. Further, in the present embodiment, the resin molded article 10 is used as a component of the apparatus 1 distributed in the market. However, the resin molded article 10 may be separately used as a product to be distributed in the market. The industrial product includes an electric-and-electronic home appliance, an electronic product such as a communication device, a transportation apparatus such as an automobile, a vehicle, and an elevator, a unit product for a kitchen and a bathroom, a construction part for a house or a building, a civil engineering part such as a road sign, an exterior housing part for a daily product, a various kind of storage container, a toy, an audio device, and a beauty appliance. More specifically, the industrial product includes parts for an electric pot, a humidifier, a rice cooker, a rice-cake making machine, a coffee maker, a dishwashing-and-drying machine, and an air conditioner, a connector housing for electric connection of automobile wire harness, and a storage tray of a semiconductor part.

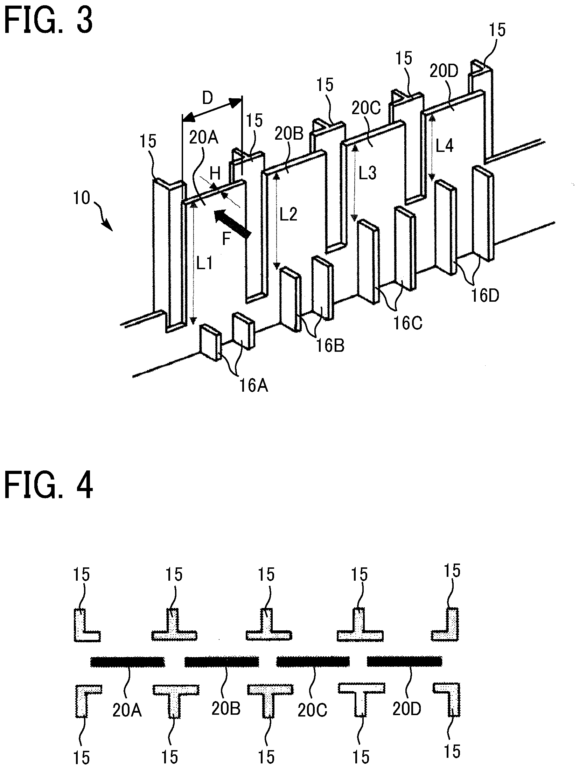

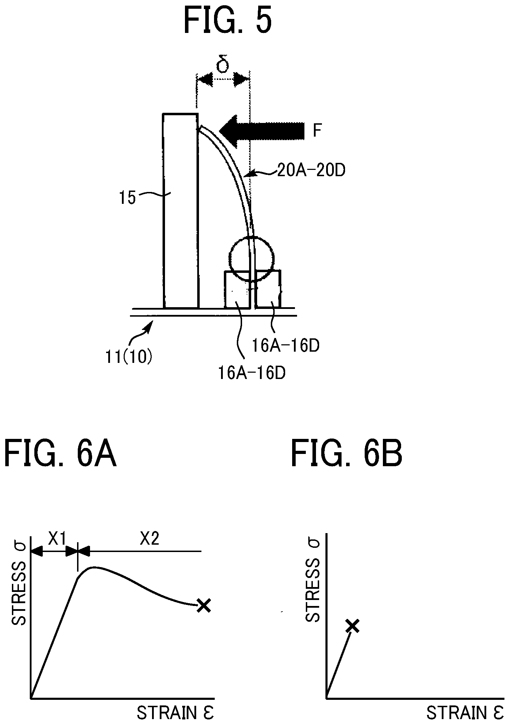

As illustrated in FIGS. 2 and 5, the resin molded article 10 according to the present embodiment includes multiple beam-shaped portions (rib-shaped portions) as parts of the resin molded article 10. However, the parts do not interfere with the original function of the resin molded article 10. The multiple beam-shaped portions (four beam-shaped portions in the present embodiment, i.e., the first beam-shaped portion 20A, the second beam-shaped portion 20B, the third beam-shaped portion 20C, and the fourth beam-shaped portion 20D) are integral parts of the resin molded article 10 such that ends on one side (that are root portions (fixed end) of the lower side in FIG. 3) are connected to the resin molded article 10 and the other ends on the other side (that are tip portions of the upper side in FIG. 3) are free ends. Specifically, the multiple beam-shaped portions (first to fourth beam-shaped portions 20A to 20D) are made of the same resin material as that of the resin molded article 10, and are formed as parts of the resin molded article 10 by the same molding process. The first to fourth beam-shaped portions 20A to 20D are formed so as to rise from a bottom portion 11 (see FIG. 5) of the resin molded article 10.

In an embodiment of the present disclosure, a resin molded article includes multiple beam-shaped portions each having a fixed end connected to a bottom of the resin molded article, and a free end rising from the bottom of the resin molded article in a first direction, the free end plastically deformable. The multiple beam-shaped portions have different maximum bending stresses in bending the free end by a predetermined amount in a second direction intersecting the first direction.

In an embodiment of the present disclosure, in the resin molded article the multiple beam-shaped portions have combinations of forms of plastically deformed, broken, plastically undeformed, and unbroken when the free end is deformed by the predetermined amount.

In an embodiment of the present disclosure, in the resin molded article, the restricting portion extends in the first direction, the restricting portion has a gap with the multiple beam-shaped portions in the second direction, and the free end of each of the multiple beam-shaped portions are deformable in the second direction.

The first to fourth beam-shaped portions 20A to 20D are formed so as to have maximum stresses ($\sigma$) different from each other when the other ends (free ends) are deformed by a predetermined amount $\delta$ (see FIG. 5). As illustrated in FIG. 3, the first to fourth beam-shaped portions 20A to 20D are formed such that at least one of the length L or the thickness H is different from each other. Further, the first to fourth beam-shaped portions 20A to 20D having a plate shape that are equal in shape (length L, thickness H, and width D) are arranged in parallel and spaced apart from each other in the width direction (a direction perpendicular to the direction to which the force F described below is applied and parallel to the width D).

In an embodiment of the present disclosure, in the resin molded article, the multiple beam-shaped portions have at least one of different lengths or different thicknesses from each other.

Specifically, in the present embodiment, the first to fourth beam-shaped portions 20A to 20D are formed so that the thickness H and the width D are the same and the lengths L1 to L4 are different from each other. The lengths L1 to L4 are lengths from the root portion (fixed end, a position connected to the bottom portion 11 of the resin molded article 10 (see FIG. 5)) to the tip portion. When the length of the first beam-shaped portion 20A at the leftmost in FIG. 3 is L1, the length of the second beam-shaped portion 20B at the right adjacent to the first beam-shaped portion 20A is L2, the length of the third beam-shaped portion 20C at the right adjacent to the second beam-shaped portion 20B is L3, and the length of the fourth beam-shaped portion 20D at the rightmost position is L4, the relation among L1 to L4 is L1 > L2 > L3 > L4. Thus, also from Equation 0 described below, when the first to fourth beam-shaped portions 20A to 20D are deformed by a predetermined amount $\delta$, the maximum stress $\sigma$ applied to the first to fourth beam-shaped portions 20A to 20D increases in inversely proportional to the square of the length L, i.e., in the order of the maximum stress $\sigma1$ of the first beam-shaped portion 20A, the maximum stress $\sigma2$ of the second beam-shaped portion 20B, the maximum stress $\sigma3$ of the third beam-shaped portion 20C, and the maximum stress $\sigma4$ of the fourth beam-shaped portion 20D (i.e., $\sigma1 < \sigma2 < \sigma3 < \sigma4$).

In the present embodiment, as illustrated in FIG. 5, the presence or absence of deterioration (strength deterioration) is determined based on the presence or absence of plastic deformation or breakage when the other ends (free ends) of the first to fourth beam-shaped portions 20A to 20D are deformed by a predetermined amount $\delta$ by applying a force F with a human finger. Specifically, when the other ends (the free ends (tip ends)) of the first to fourth beam-shaped portions 20A to 20D are deformed by a predetermined amount $\delta$, some of the other ends are plastically deformed (plastic deformation), broken (breakage), or not changed (no-change). The degree of deterioration of the resin molded article 10 is evaluated by a combination of the plastic deformation, breakage, and no-change, and the reuse of the resin- molded article 10 is determined by the degree of the deterioration.

In other words, the first to fourth beam-shaped portions 20A to 20D of the resin molded article 10 in a disassembled state are deformed in the direction of the force F (represented by the arrow in FIG. 5) in a recycling factory, and the strength of the resin molded article 10 is evaluated based on whether plastic deformation or breakage has occurred in any of the first to fourth beam-shaped portions 20A to 20D.

More specifically, as a method for determining whether the resin molded article 10 has deteriorated, a deforming step that deforms the other ends (free ends) of the first to fourth beam-shaped portions 20A to 20D is performed. As described above, based on the combination of the plastic deformation, breakage, and non-change occurred in the first to fourth beam-shaped portions 20A to 20B in the deforming step, a determining step that evaluates deterioration occurred in the resin molded article 10 is performed. Based on the determination in the determining step, the resin molded article 10 is determined to be reusable or not.

For example, the first beam-shaped portion 20A has the length L1 (maximum stress $\sigma1$) that is set so as to be plastically deformed or broken at a strength (bending stress) at which the resin molded article 10 cannot be reused. The second beam-shaped portion 20B has the length L2 (maximum stress $\sigma2$) that is set so as to be plastically deformed or broken at a minimum necessary strength (bending stress) to reuse the resin molded article 10. The third beam-shaped portion 20C has the length L3 (maximum stress $\sigma3$) that is set so as to be plastically deformed or broken at twice the minimum necessary strength (bending stress) to reuse the resin molded article 10. The third beam-shaped portion 20C has the length L3 (maximum stress $\sigma4$) that is set so as to be plastically deformed or broken at three times the minimum necessary strength (bending stress) to reuse the resin molded article 10.

When the other ends (free ends) of the first to fourth beam-shaped portions 20A to 20D are deformed by a predetermined amount $\delta$ (in the deforming step), the first and second beam-shaped portions 20A and 20B are not plastically deformed or broken, and the third and fourth beam-shaped portions 20C and 20D are plastically deformed or broken, the resin molded article 10 is determined to be reusable as the resin molded article 10 has the minimum necessary strength to reuse (i.e., the resin molded article 10 does not have significant deterioration). By contrast, when the first beam-shaped portion 20A is not plastically deformed or broken, and the second to fourth beam-shaped portions 20B to 20D are plastically deformed or broken, the resin molded article 10 is determined not to be reusable as the resin molded article 10 does not have the minimum necessary strength to reuse (i.e., the resin article molded 10 has deteriorated). Further, when the first to fourth beam-shaped portion 20A to 20D are plastically deformed or broken, the resin molded article is determined not to be reusable as the resin molded article 10 does not have the minimum necessary strength to reuse (i.e., the resin molded article 10 has deteriorated). By contrast, when the first to fourth beam-shaped portion 20A to 20D are not plastically deformed or broken, the resin molded article is determined to be reusable as the resin molded article 10 has a sufficient strength to reuse (i.e., the resin molded article 10 has almost no deterioration). Thus, based on these determinations, a resin molded article 10 that is deteriorated and does not have a sufficient strength is not reused, and a resin molded article 10 that is not deteriorated and has a sufficient strength is reused.

As described above, in the resin molded article 10 according to the present embodiment, the deterioration of the resin molded article 10 can be determined by deforming the first to fourth beam-shaped portions 20A to 20D that are integral parts of the resin molded article 10 without using a measuring device, and the determination operation can be performed in a simple manner.

As illustrated in FIGS. 3 to 5, the resin molded article 10 according to the present embodiment includes a restricting portion 15 that restricts the deformation amount of the other ends (free ends) of the first to fourth beam-shaped portions 20A to 20D when the other ends are deformed. The restricting portion 15 is formed in a substantially plate shape so as to extend in the direction in which the first to fourth beam-shaped portions 20A to 20D rise and at intervals with respect to the first to fourth beam-shaped portions 20A to 20D in a state in which the other ends of the first to fourth beam-shaped portions 20A to 20D are not deformed (in a state in which the force F (external force) is not applied). Specifically, the restricting portion 15 is made of the same resin material as that of the resin molded article 10, and is formed as a part of the resin molded article 10 by the same molding process. The restricting portion 15 is formed so as to rise from the bottom portion 11 (see FIG. 5) of the resin molded article 10. The "deforming step" described above is a step that deforms the first to fourth beam-shaped portions 20A to 20D to a position at which the other ends of the first to fourth beam-shaped portions 20A to 20D abut on the restricting portion 15. When the force F (external force or load) is applied to the first to fourth beam-shaped portions 20A to 20D to deform, the restricting portion 15 allows deformation of the first to fourth beam-shaped portions 20A to 20D to a predetermined position (i.e., position that abuts on the restricting portion 15). As a result, the deterioration can be stably determined.

In an embodiment of the present disclosure, the resin molded article further includes a restricting portion to restrict a deformation amount of the free end of each of the multiple beam-shaped portions.

As illustrated in FIG. 4, the restricting portion 15 can be disposed on both sides (upper and lower sides in FIG. 4) such that the first to fourth beam-shaped portions 20A to 20D are disposed between the restricting portions 15. In this case, the first to fourth beam-shaped portions 20A to 20D may be deformed to any of the two sides at the time when deterioration is determined. As illustrated in FIG. 4, it is preferable that the multiple restricting portions 15 are disposed at intervals in the width direction (i.e., the right and left direction in FIG. 4) while obtaining a gap for applying the force F (load) to the first to fourth beam-shaped portions 20A to 20D.

As illustrated in FIGS. 3 and 5, multiple reinforcing portions (first to fourth reinforcing portions 16A to 16D in the present embodiment) that reinforce the root portion (fixed end) of ends of the first to fourth beam-shaped portions 20A to 20D of the resin molded article 10 on one side in the present disclosure are disposed so as to restrict elastic deformation of the root portion (fixed end). The first to fourth reinforcing portions 16A to 16D are disposed at positions in contact with the root portion (fixed end) of the first to fourth beam-shaped portions 20A to 20D, and have a thickness (thickness in the longitudinal direction in FIG. 5) that is thicker than the thickness of the surroundings. Specifically, the first to fourth reinforcing portions 16A to 16D are made of the same resin material as that of the resin molded article 10, and are formed as parts of the resin molded article 10 by the same molding process. The first to fourth reinforcing portions 16A to 16D are formed so as to rise from the bottom portion 11 (see FIG. 5) of the resin molded article 10. The first to fourth beam-shaped portions 20A to 20D in the present embodiment are formed so that the lengths L1 to L4 are different from each other. Since the positions of the root portions (fixed ends) are different from each other, the lengths of the first to fourth reinforcing portions 16A to 16D (the lengths in the same direction as the lengths L1 to L4 of the first to fourth beam-shaped portions 20A to 20D) are also different from each other. When the force F (external force) is applied to the first to fourth beam-shaped portions 20A to 20D to deform, the first to fourth reinforcing portions 16A to 16D allow the first to fourth beam-shaped portions 20A to 20D to be elastically deformed at portions over the first to fourth reinforcing portions 16A to 16D (i.e., variations are less likely to occur in elastically deformed portions). As a result, the deterioration can be stably determined. As illustrated in FIG. 5, the first to fourth reinforcing portions 16A to 16D can be disposed on both sides of the first to fourth beam-shaped portions 20A to 20D so as to hold the first to fourth beam-shaped portions 20A to 20D between the first to fourth reinforcing portions 16A to 16D (the left side and the right side of the first to fourth beam-shaped portions 20A to 20D in FIG. 5). In this case, the above-described function of the first to fourth reinforcing portion 16A to 16D is likely to exhibit.

In an embodiment of the present disclosure, the resin molded article further includes multiple reinforcing portions adjacent to the fixed end of each of the multiple beam-shaped portions on the bottom of the resin molded article. The multiple reinforcing portions restrict an elastic deformation of the fixed end.

In an embodiment of the present disclosure, in the resin molded article, the multiple reinforcing portions are in contact with the fixed end, and the multiple reinforcing portions have a thickness thicker than a thickness of surroundings of the multiple reinforcing portions in the first direction.

In an embodiment of the present disclosure, in the resin molded article a determining method includes deforming a free end of each of multiple beam-shaped portions of a resin molded article. The free end of each of the multiple beam-shaped portions is plastically deformable or breakable. The multiple beam-shaped portions have combinations of form of plastically deformed, broken, plastically undeformed, and unbroken when the free end is deformed by a predetermined amount.

In an embodiment of the present disclosure, the resin molded article further includes determining the free end of each of the multiple beam-shaped portions plastically deformed or broken, and reusing the resin molded article based on the determining.

In the "deforming step" described above, the first to fourth beam-shaped portions 20A to 20D are deformed by a predetermined amount $\delta$ under the restriction of the restricting portion 15, and the maximum stress $\sigma$ applied to the first to fourth beam-shaped portions 20A to 20D can be obtained by Equation 0 below based on the calculation equation of the deflection of the cantilever beam of the material mechanics.

$$\sigma = 3\,E\,H\,\delta\,/\,(2\,L^2) \quad \text{(Equation 0)}$$

where E, H, and L represent the longitudinal elastic modulus, the thickness (see FIG. 3), and the length (see FIG. 3) of the first to fourth beam-shaped portions 20A to 20D, respectively. Equation 0 is obtained from Equations 1 to 3 below. Equation 1 obtains the deformation $\delta$, Equation 2 obtains the maximum bending moment Mmax, and Equation 3 obtains the maximum stress $\delta$max.

$$\delta = F\,L^3\,/\,(3\,E\,I) \quad \text{(Equation 1)}$$

$$Mmax = F\,L \quad \text{(Equation 2)}$$

$$\sigma\,max = Mmax\,/\,Z \quad \text{(Equation 3)}$$

In Equations 1 to 3, F represents a load applied to the beam-shaped portion 20 (see FIG. 3), I represents a second moment of area (= $D\,H^3\,/\,12$), D represents a width (see FIG. 3), and Z represents a section modulus (= $D\,H^2\,/\,6$).

As can be understood from equations, the applied maximum stress $\sigma$ that deforms the first to fourth beam-shaped portions 20A to 20D by a predetermined amount $\delta$ is a value determined by the longitudinal elastic modulus E (a value that has a small change even if the material deteriorates with elapsed time), the thickness H of the first to fourth beam-shaped portions 20A to 20D (a value that does not change with elapsed time), and the length L of each of the first to fourth beam-shaped portions 20A to 20D (a value that does not change with elapsed time). Thus, since the value of the width D of the first to fourth beam-shaped portions 20A to 20D is not related to the maximum stress $\sigma$, the deterioration determination can be performed with equivalent accuracy for all the first to fourth beam-shaped portions 20A to 20D disposed at the resin molded article 10 as long as the thickness H and the length L are equal, even if the widths D are different. In other words, the maximum stress $\sigma$ of the first to fourth beam-shaped portions 20A to 20D varies when either the thickness H or the length L is different, regardless of the difference in the width D. In the present embodiment, the first to fourth beam-shaped portions 20A to 20D having different lengths L and different maximum stresses $\sigma$ among the thickness H and the length L are deformed by a predetermined amount $\delta$, respectively, to determine the degree of deterioration of the resin molded article 10. Thus, deterioration can be determined with high accuracy without using a measuring device.

FIGS. 6A and 6B are graphs of the relation between strain $\varepsilon$ and stress $\sigma$ in the first to fourth beam-shaped portions 20A to 20D. FIG. 6A is a graph of an initial state in which the resin molded article 10 is not deteriorated, and FIG. 6B is a graph of an elapsed state in which the resin molded article 10 has deteriorated. As illustrated in FIG. 6A, in the initial state, the resin molded article 10 is not deteriorated in strength. When a force is applied to the resin molded article 10 (beam-shaped portion 20), the resin molded article 10 is elastically deformed to a certain stress (range X1), and when the force exceeds the certain stress, the resin molded article 10 is plastically deformed. When the deformation is further increased, the resin molded article 10 is fractured (range X2). The stress at the time of plastic deformation or fracture can be basically regarded as the strength of the material (resin molded article 10). By contrast, as illustrated in FIG. 6B, when the strength of the resin molded article 10 deteriorates due to, for example, a decrease in the molecular weight of the resin material caused by heat, or ultraviolet rays, or chemicals with elapsed time, the longitudinal elastic modulus (the slope of the graph in the elastic region) hardly changes. However, deterioration such as sudden fracture with almost no plastic deformation is likely to occur with a stress smaller than that in a state where there is no deterioration. The strength of the material (resin molded article 10) at this time is smaller than the strength in the state without deterioration (state illustrated in FIG. 6A).

In an embodiment of the present disclosure, the stress at a time when the tips of the first to fourth beam-shaped portions 20A to 20D are deformed by applying the force F until the first to fourth beam-shaped portions 20A to 20D abut on the restricting portion 15 do not change before and after the deterioration as described above. When the shape of the beam-shaped portion 20 such as the thickness H and the length L is set such that the maximum stress $\sigma$ at the time when the first to fourth beam-shaped portions 20A to 20D are deformed by a predetermined amount $\delta$ becomes a threshold value in the determination of the material strength of the resin molded article 10, the strength deterioration can be evaluated by the presence or absence of plastic deformation or breakage at the time when the same stress F is applied to the first to fourth beam-shaped portions 20A to 20D. Thus, even when the strength deterioration of the resin molded article 10 caused by a decrease in molecular weight due to temperature, humidity, or ultraviolet rays occurs with elapsed time, the strength deterioration can be simply determined by observing the presence or absence of plastic deformation or fracture at the time when the tip of the first to fourth beam-shaped portions 20A to 20D is deformed by applying the force F until the first to fourth beam-shaped portions 20A to 20D abut on the restricting portion 15, without using a measuring device and without requiring the skill of an operator. In the present embodiment, the first to fourth beam-shaped portions 20A to 20D having different maximum stresses $\delta$ are disposed, but the number of beam-shaped portion is not limited to four, and may be two, three, or five or more.

First Modification

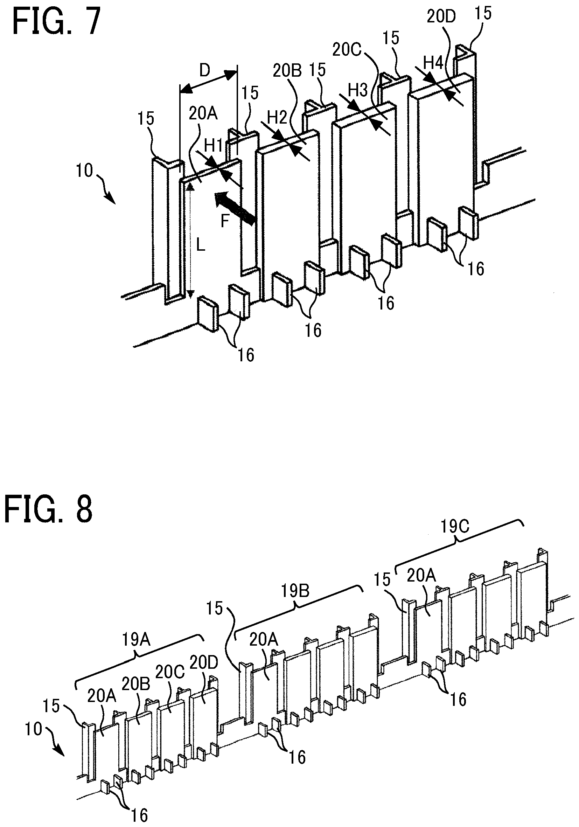

As illustrated in FIG. 7, the resin molded article 10 in the first modification differs from the resin molded article 10 illustrated in FIG. 3 in that the first to fourth beam-shaped portions 20A to 20D are formed so as to have the same length L and width D and different thicknesses H1 to H4. When the thickness of the first beam-shaped portion 20A at the leftmost in FIG. 7 is H1, the thickness of the second beam-shaped portion 20B at the right adjacent to the first beam-shaped portion 20A is H2, the thickness of the third beam-shaped portion 20C at the right adjacent to the first beam-shaped portion 20B is L3, and the thickness of the fourth beam-shaped portion 20D at the rightmost is H4, the relation among the H1 to H4 is H1 < H2 < H3 < H4. Thus, also from Equation 0 described above, when the first to fourth beam-shaped portions 20A to 20D are deformed by a predetermined amount $\delta$, the maximum stress $\sigma$ applied to the first to fourth beam-shaped portions 20A to 20D increases in the order of the maximum stress $\sigma$1 of the first beam-shaped portion 20A, the maximum stress $\sigma$2 of the second beam-shaped portion 20B, the maximum stress $\sigma$3 of the third beam-shaped portion 20C, and the maximum stress $\sigma$4 of the fourth beam-shaped portion 20D in proportion to the thickness H ($\sigma$1 < $\sigma$2 < $\sigma$3 < $\sigma$4). In the case of such a configuration, the deterioration of the resin molded article 10 can be determined by the determining method for the resin molded article 10 as described above, and the possibility of reusing the resin molded article 10 can be determined. The first to fourth beam-shaped portions 20A to 20D having different lengths L1 to L4 are used in the example of FIG. 3, and the first to fourth beam-shaped portions 20A to 20D having different thicknesses H1 to H4 are used in the example of FIG. 7. Further, the first to fourth beam-shaped portions 20A to 20D having different lengths L1 to L4 and different thicknesses H1 to H4 may be used.

Second Modification

As illustrated in FIG. 8, in the resin molded article 10 according to the second modification, multiple groups of multiple beam-shaped portions (i.e., a first beam-shaped portion group 19A, a second beam-shaped portion group 19B, and a third beam-shaped portion group 19C) are disposed. The first beam-shaped portion group 19A include the first to fourth beam-shaped portions 20A to 20D described above with reference to FIG. 7 (or FIG. 3). Similarly, the second beam-shaped portion group 19B includes the first to fourth beam-shaped portions 20A to 20D described above with reference to FIG. 7 (or FIG. 3) at the right adjacent to the first beam-shaped portion group 19A at an interval. Further, the third beam-shaped portion group 19C also includes the first to fourth beam-shaped portions 20A to 20D described above with reference to FIG. 7 (or FIG. 3) at the right adjacent to the second beam-shaped portion group 19B at an interval. When the resin molded article 10 is reused (to be reused), the above-described deforming step is performed on any one of the first to third beam-shaped portion groups 19A to 19C. The number of times of reuse can be counted by the number of the beam-shaped portion group that have deformed among the first to third beam-shaped portion groups 19A to 19C. In other words, the worker who determines the deterioration and the worker who assembles the apparatus 1 using the reused resin molded article 10 can grasp the number of times of reuse of the resin molded article 10 from the number of the beam-shaped portion group on which the deforming step has performed among the beam-shaped portion groups 19A to 19C. As a result, the workability of the worker is increased. In the second modification, the first to third beam-shaped portion groups 19A to 19C are used, but the number of the beam-shaped portion group is not limited to three, two or four or more beam-shaped portion groups may be used.

In an embodiment of the present disclosure, the resin molded article further includes multiple groups of the multiple beam-shaped portions.

As described above, the resin molded article 10 according to the present embodiment is a resin molded article made of a predetermined resin material, and the first to fourth beam-shaped portions 20A to 20D are integral parts of the resin molded article 10 such the ends of the first to fourth beam-shaped portions 20A to 20D on one side are connected to the resin molded article 10 and the other ends of the first to fourth beam-shaped portions 20A to 20D are free ends. The first to fourth beam-shaped portions 20A to 20D are formed such that each of the first to fourth beam-shaped portions 20A to 20D has a maximum stress $\sigma$ different from each other when the other end (free end) is deformed by a predetermined amount $\delta$. The presence or absence of deterioration is determined by the presence or absence of plastic deformation or breakage when the other ends of the first to fourth beam-shaped portions 20A to 20D are deformed by a predetermined amount δ. Accordingly, the deterioration of the resin molded article 10 can be determined in a simple manner.

In an embodiment of the present disclosure, the resin molded article further includes forming multiple groups of the multiple beam-shaped portions to deform a group of the multiple groups, and counting a number of the reusing from a number of the group of the multiple groups having the free end deformed.

It is obvious that the present disclosure is not limited to the present embodiment, and the present embodiment can be modified as appropriate in addition to the modifications suggested in the present embodiment within the scope of the technical idea of the present disclosure. In addition, the number, position, and shape of the constituent members described above are not limited to those in the present embodiment, and can be set to the number, position, or shape preferable for conducting the present embodiment.

Aspects of the present disclosure are, for example, as follows.

First Aspect

A resin molded article made of a predetermined resin material includes multiple beam-shaped portions including one ends connecting to the resin molded article as one body and other ends as free ends on another side. The deterioration of the resin molded article is determined by presence or absence of plastic deformation or breakage at a time when other ends of the multiple beam-shaped portions are deformed by a predetermined amount.

Second Aspect

In the resin molded article according to the first aspect, when the other ends of the multiple beam-shaped portions are deformed by a predetermined amount, some of the other ends are plastically deformed (plastic deformation), broken (breakage), or not changed (no-change). The degree of deterioration of the resin molded article is evaluated by a combination of the plastic deformation, breakage, and no-change, and the reuse of the resin molded-article is determined by the degree of the deterioration.

Third Aspect

The resin molded article according to the first or second aspect, further includes a restricting portion to restrict a deformation amount of the other ends at a time when the other ends of the beam-shaped portion are deformed.

Fourth Aspect

In the resin molded article according to the third aspect, the restricting portion is formed so as to extend in a direction in which the multiple beam-shaped portions rise at an interval with respect to multiple the beam-shaped portions in a state where the other ends of the multiple beam-shaped portions are not deformed.

Fifth Aspect

The resin molded article according to any one of the first to fourth aspects further includes multiple reinforcing portions to reinforce a root portion of the multiple beam-shaped portions so as to restrict elastic deformation.

Sixth Aspect

In the resin molded article according to the fifth aspect, the multiple reinforcing portions are disposed at positions in which the multiple reinforcing portion are in contact with the root portion, and thicknesses of the multiple reinforcing portions are thicker than a thickness of surroundings of the multiple reinforcing portions.

Seventh Aspect

In the resin molded article according to any one of the first to seventh aspects, the multiple beam-shaped portions have the at least one of the same length or thickness.

Eighth Aspect

The resin molded article according to any one of the first to seventh aspects further includes multiple groups of the multiple beam-shaped portions.

Ninth Aspect

In a determining method for deterioration of a resin molded article made of a predetermined resin material, the resin molded article includes multiple beam-shaped portions including ends on one side connecting to the resin molded article as one body and other ends as free ends on another side. The multiple beam-shaped portions are formed so as to have different maximum stresses each other, and when the other ends of the multiple beam-shaped portions are deformed by a predetermined amount, some of the other ends are plastically deformed (plastic deformation), broken (breakage), or not changed (no-change). The determining method includes deforming the other ends of the multiple beam-shaped portions by a predetermined amount, and determining the degree of deterioration of the resin molded article based on a combination of the plastic deformation, breakage, and no-change.

Tenth Aspect

The determining method according to the ninth aspect further includes excluding the resin molded article determined to have deteriorated in the determining from reuse.

Eleventh Aspect

In the determining method according to ninth or tenth aspect, the resin molded article include multiple groups of the multiple beam-shaped portions. When the resin molded article is reused, the deforming is performed on one group of the multiple beam-shaped portions of the multiple groups of the multiple beam-shaped portions. The determining method further includes counting the number of the multiple groups of the multiple beam-shaped portions that have been deformed.

Twelfth Aspect

A resin molded article includes multiple beam-shaped portions each having a fixed end connected to a bottom of the resin molded article, and a free end rising from the bottom of the resin molded article in a first direction, the free end plastically deformable. The multiple beam-shaped portions have different maximum bending stresses in bending the free end by a predetermined amount in a second direction intersecting the first direction.

Thirteenth Aspect

In the resin molded article according to the twelfth aspect, the multiple beam-shaped portions have combinations of forms of plastically deformed, broken, plastically undeformed, and unbroken when the free end is deformed by the predetermined amount.

Fourteenth Aspect

The resin molded article according to the twelfth or thirteenth aspect further includes a restricting portion to restrict a deformation amount of the free end of each of the multiple beam-shaped portions.

Fifteenth Aspect

In the resin molded article according to fourteenth aspect, the restricting portion extends in the first direction, the restricting portion has a gap with the multiple beam-shaped portions in the second direction, and the free end of each of the multiple beam-shaped portions are deformable in the second direction.

Sixteenth Aspect

The resin molded article according to any one of the twelfth to fifteenth aspects further includes multiple reinforcing portions adjacent to the fixed end of each of the multiple beam-shaped portions on the bottom of the resin molded article. The multiple reinforcing portions restrict an elastic deformation of the fixed end.

Seventeenth Aspect

In the resin molded article according to the sixteenth aspect, the multiple reinforcing portions are in contact with the fixed end, and the multiple reinforcing portions have a thickness thicker than a thickness of surroundings of the multiple reinforcing portions in the first direction.

Eighteenth Aspect

In the resin molded article according to any one of the twelfth to seventeenth aspects, the multiple beam-shaped portions have at least one of different lengths or different thicknesses from each other.

Nineteenth Aspect

The resin molded article according to any one of the twelfth to eighteenth aspects further includes multiple groups of the multiple beam-shaped portions.

Twentieth Aspect

A determining method includes deforming a free end of each of multiple beam-shaped portions of a resin molded article. The free end of each of the multiple beam-shaped portions is plastically deformable or breakable. The multiple beam-shaped portions have combinations of form of plastically deformed, broken, plastically undeformed, and unbroken when the free end is deformed by a predetermined amount.

Twenty-first Aspect

The determining method according to the twentieth aspect further includes determining the free end of each of the multiple beam-shaped portions plastically deformed or broken, and reusing the resin molded article based on the determining.

Twenty-second Aspect

The determining method according to the twentieth or twenty-first aspect further includes forming multiple groups of the multiple beam-shaped portions to deform a group of the multiple groups, and counting a number of the reusing from a number of the group of the multiple groups having the free end deformed.
The above-described embodiments are illustrative and do not limit the present invention. Thus, numerous additional modifications and variations are possible in light of the above teachings. For example, elements and/or features of different illustrative embodiments may be combined with each other and/or substituted for each other within the scope of the present invention. Any one of the above-described operations may be performed in various other ways, for example, in an order different from the one described above.

The functionality of the elements disclosed herein may be implemented using circuitry or processing circuitry which includes general purpose processors, special purpose processors, integrated circuits, ASICs ("Application Specific Integrated Circuits"), FPGAs ("Field-Programmable Gate Arrays"), and/or combinations thereof which are configured or programmed, using one or more programs stored in one or more memories, to perform the disclosed functionality. Processors are considered processing circuitry or circuitry as they include transistors and other circuitry therein. In the disclosure, the circuitry, units, or means are hardware that carry out or are programmed to perform the recited functionality. The hardware may be any hardware disclosed herein which is programmed or configured to carry out the recited functionality.

**Claims**

1. A resin molded article (10) comprising:
   multiple beam-shaped portions (20A to 20D) each having:

   > a fixed end connected to a bottom of the resin molded article (10); and
   > a free end rising from the bottom of the resin molded article in a first direction, the free end plastically deformable,

   wherein the multiple beam-shaped portions (20A to 20D) have different maximum bending stresses in bending the free end by a predetermined amount in a second direction intersecting the first direction.

2. The resin molded article (10) according to claim 1,

   > wherein the multiple beam-shaped portions (20A to 20B) have combinations of forms of:

   > > plastically deformed;
   > > broken;
   > > plastically undeformed; and
   > > unbroken,

   > when the free end is deformed by the predetermined amount.

3. The resin molded article (10) according to claim 1 or 2, further comprising a restricting portion (15) to restrict a deformation amount of the free end of each of the multiple beam-shaped portions (20A to 20D).

4. The resin molded article (10) according to claim 3, wherein the restricting portion (15) extends in the first direction,

   > the restricting portion (15) has a gap with the multiple beam-shaped portions (20A to 20D) in the second direction, and
   > the free end of each of the multiple beam-shaped portions (20A to 20D) are deformable in the second direction.

5. The resin molded article (10) according to claim 1 or 2, further comprising multiple reinforcing portions (16A to 16D) adjacent to the fixed end of each of the multiple beam-shaped portions (20A to 20D) on the bottom of the resin molded article (10),
   wherein the multiple reinforcing portions restrict an elastic deformation of the fixed end.

6. The resin molded article according to claim 5,

   > wherein the multiple reinforcing portions (16A to 16D) are in contact with the fixed end,
   > the multiple reinforcing portions (16A to 16D) have a thickness thicker than a thickness of surroundings of the multiple reinforcing portions (16A to 16D) in the first direction.

7. The resin molded article according to claim 1 or 2, wherein the multiple beam-shaped portions (20A to 20D) have at least one of different lengths or different thicknesses from each other.

8. The resin molded article according to claim 1 or 2, further comprising multiple groups (19A to 19C) of the multiple beam-shaped portions (20A to 20D).

9. A determining method comprising:

   > deforming a free end of each of multiple beam-shaped portions of a resin molded article,

   > > the free end of each of the multiple beam-shaped portions being plastically deformable or breakable,
   > > wherein the multiple beam-shaped portions have combinations of form of:

   > > > plastically deformed;
   > > > broken;
   > > > plastically undeformed; and
   > > > unbroken,

   > when the free end is deformed by a predetermined amount.

**10.** The determining method according to claim 9, further comprising:
determining the free end of each of the multiple beam-shaped portions plastically deformed or broken; and
reusing the resin molded article based on the determining.

**11.** The determining method according to claim 10, further comprising:
forming multiple groups of the multiple beam-shaped portions to deform a group of the multiple groups; and
counting a number of the reusing from a number of the group of the multiple groups having the free end deformed.

FIG. 1

FIG. 2

FIG. 3

FIG. 4

# FIG. 5

# FIG. 6A

# FIG. 6B

## FIG. 7

## FIG. 8

| | Europäisches<br>Patentamt<br>European<br>Patent Office<br>Office européen<br>des brevets | **EUROPEAN SEARCH REPORT** | Application Number<br><br>EP 24 18 5264 |
|---|---|---|---|

## DOCUMENTS CONSIDERED TO BE RELEVANT

| Category | Citation of document with indication, where appropriate,<br>of relevant passages | Relevant<br>to claim | CLASSIFICATION OF THE<br>APPLICATION (IPC) |
|---|---|---|---|
| Y | WO 2021/106680 A1 (MURATA MANUFACTURING CO<br>[JP]) 3 June 2021 (2021-06-03)<br>* ref.1,4 *<br>- - - - - | 1-5 | INV.<br>B29C45/00<br>G01N3/08<br>G01N33/44 |
| Y | JP 2001 180782 A (TOSHIBA CORP)<br>3 July 2001 (2001-07-03)<br>* par.25 *<br>- - - - - | 1-5 | ADD.<br>B29L31/40 |

**TECHNICAL FIELDS<br>SEARCHED (IPC)**

B29C<br>G01N<br>B29L

The present search report has been drawn up for all claims

| Place of search | Date of completion of the search | Examiner |
|---|---|---|
| Munich | 5 November 2024 | Tonelli, Enrico |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons
...........................................................................
& : member of the same patent family, corresponding
  document

EPO FORM 1503 03.82 (P04C01)

## ANNEX TO THE EUROPEAN SEARCH REPORT
## ON EUROPEAN PATENT APPLICATION NO.

EP 24 18 5264

This annex lists the patent family members relating to the patent documents cited in the above-mentioned European search report.
The members are as contained in the European Patent Office EDP file on
The European Patent Office is in no way liable for these particulars which are merely given for the purpose of information.

05-11-2024

| Patent document cited in search report | Publication date | Patent family member(s) | | Publication date |
|---|---|---|---|---|
| WO 2021106680 A1 | 03-06-2021 | CN | 114728455 A | 08-07-2022 |
| | | JP | WO2021106680 A1 | 03-06-2021 |
| | | US | 2022242020 A1 | 04-08-2022 |
| | | WO | 2021106680 A1 | 03-06-2021 |
| JP 2001180782 A | 03-07-2001 | JP | 3998383 B2 | 24-10-2007 |
| | | JP | 2001180782 A | 03-07-2001 |

EPO FORM P0459

For more details about this annex : see Official Journal of the European Patent Office, No. 12/82

**EP 4 491 366 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001180782 A

- JP 2013029371 A